# EUROPEAN PATENT SPECIFICATION

(11) **EP 1 840 205 B1**
(45) Date of publication and mention of the grant of the patent: **08.04.2009**
(21) Application number: 05818112.4
(22) Date of filing: 03.11.2005
(51) Int. Cl.: C12N 1/20, A23C 9/12, A61K 35/74, A23L 1/29, A61K 8/99

(54) **BIFIDOBACTERIUM LACTIS 668 STRAIN USED AS A COMPONENT FOR FOOD PRODUCTS, STARTERS, MEDICINAL AND COSMETIC AGENTS**
ALS BESTANDTEIL VON LEBENSMITTELPRODUKTEN, STARTERN, MEDIZINISCHEN UND KOSMETISCHEN MITTELN VERWENDETER BIFIDOBACTERIUM LACTIS 668-STAMM
SOUCHE DE BIFIDOBACTERIUM LACTIS 668 UTLISEE DANS DES FERMENTS, DES PRODUITS ALIMENTAIRES, DES MEDICAMENTS ET DES PRODUITS COSMETIQUES

(30) Priority: 31.12.2004 RU 2004138993
(43) Date of publication of application: 03.10.2007
(73) Proprietor: Open Joint Stock Company "Wimm-Bill-Dann", Moscow 127591 (RU)
(72) Inventor: VUSTINA, Tatiana Fedorovna, Moscow, 115612 (RU); PERMINOV, Sergey Igorevich, St.Petersburg, 197210 (RU); MOZGOVAYA, Irina Nikolaevna, Kaluga, 248031 (RU)
(74) Representative: Benatov, Emil Gabriel
(86) International application number: PCT/RU2005/000545
(87) International publication number: WO 2006/073329

(56) References cited:
- WO-A-99/10476
- WO-A1-01/97822
- WO-A1-03/099037
- RU-C1- 2 215 028
- PRODUCT DESCRIPTION-Bf. Species 420 DIRECT FP 50 u V7. Art. No. 13581032, DANISKO. 29.04.2003
- SHMID K. ET AL.: 'Biofidoshtamm dlya ukrepleniya immunnoi sistemy' MOLOCHNAYA PROMYSHLENNOST no. 4, 2004, page 44, XP008094694

## Description

### Field of the Invention

The invention relates to new bifidum bacteria Bifidobacterium lactis 668 strain, VKPM Ac-1673. The inventive strain is particularly used for making cultured-milk, fermented and non-fermented food products, starters, makeup preparations, biologically active additives and bacterial preparations.

### Prior Art

The unfavorable ecological environment as well as prolonged and, sometimes, irregular antibiotic therapy increase the number of people and animals suffering from disorders of the normal organism microflora. In the treatment and prophylaxis of such disorders ever growing attention is paid to both preparations having pro-biotic action and food products containing bifidus bacteria.

Food products containing bifidus bacteria are an efficient means for prophylaxis and treatment of various gastrointestinal disorders as well as intestinal dysbacterioses caused by old intestinal and other diseases, prolonged antibiotic therapy, radiation therapy chemotherapy, exposure to stresses, the unfavorable environment and other factors.

The stability of bacteria to the conditions existing in the gastrointestinal tract is an essential criterion for selecting strains contributing to health improvement.

The quality of treatment and prophylaxis products and preparations is conditioned to a large extent by the properties of a producer strain, its specific activity and technological effectiveness. A great assortment of biologically active and technologically effective strains of bifidus bacteria enables development of new mono products and complex products for dietotherapy and bacterial preparations capable of ensuring a high resistance to colony formation in the bowels of human and animal beings, restore their normal microflora and reduce occurrence of enteric infection cases.

Foreign mono preparations and complex preparations - eubiotics and cultured-milk foods - are known, which compositions comprise bifidus bacteria of the following types: Bifidobacterium bifidum (FR Patent 2336886, 29.07.1977, A 23 C 9/12), Bifidobacterium bifidum, Bifidobacterium longum, Bifidobacterium adolescentis (FR Patent 2411008, 06.07.1979, A 61 K 35/74), Bifidobacterium longum, Bifidobacterium breve (CH Patent 637297, 29.07.1983, A 61 K 39/02), Bifidobacterium lactis (US Patent 6379663, 14.03.2002, A 01 N 63/00).

For making bacterial preparations and dietotherapy products in our country the following types of bifidus bacterium strains are used: Bifidobacterium bifidum, VKPM Ac-1578 (RU Patent 2152993, 20.07.2000, C 12 N 01/20), Bifidobacterium bifidum, VKPM S-1257 (RU Patent 2108383, 10.04.1998, C 12 N 01/20), Bifidobacterium longum V379M, Bifidobacterium bifidum 791 (RU Patent 2092064 10.10.1997, A 23 C 09/12), Bifidobacterium adolescentis MS-42 (Inventor's Certificate SU 863639, 15.09.1981, C 12 N 15/00). For making cultured-milk foods enriched with bifidus bacteria foreign strains are used also, e.g., Bifidobacterium lactis HN-019 (RU Patent 2208632. 20.07.2003, C 12 N 01/20), Bifidobacterium lactis 420 (Specification for Strain, Bifidobacterium lactis 420 of DANISCO Company, dated 02.05.2003).

Nothing is known so far about using domestic strains of Bifidobacterium lactis in the above fields.

The closest to the inventive strain is the strain of Bifidobacterium lactis 420 (prototype) (Specification for Strain, Bifidobacterium lactis 420 of DANISCO Company, dated 02.05.2003). This strain has antagonistic activity in relation to some pathogenic and conditionally pathogenic microorganisms.

In a number of its checked properties the known strain of Bifidobacterium lactis 420 is inferior to the inventive strain of Bifidobacterium lactis 668; thus, in particular, it is more sensitive to phenol and bile, less active in relation to some pathogenic microorganisms (see Tables 1 and 2).

### Description of the Invention

The objective of this invention is to obtain a microorganism strain of Bifidobacterium lactis type for the purpose of using it in making cultured-milk, fermented and non-fermented foods, starters, biologically active additives, bacterial preparations, makeup preparations, and other products containing bifidus bacteria.

By achieving the said objective the following technical effects are obtained: the strain has high biological activity, in particular high anti-oxidant activity (superoxide dismutase activity), compared to the information available from the literature, stability to environmental conditions, it quickly propagates on artificial nutritional media, has acid-forming and antagonistic activity in relation to pathogenic and conditionally pathogenic microfloras. The obtaining of strains having similar properties contributes to enlarging the assortment of pro-biotics used for health support, in particular for normalizing the microflora of the gastrointestinal tract and the urogenital tract, the cutaneous coverings and the mucilaginous coverings of human and animal beings.

The stated objective is achieved through using the Bifidobacterium lactis 668 strain (Ac-1673 VKPM) in compositions of starters, foods, medical, bacterial, makeup preparations, etc.

The Bifidobacterium lactis 668 strain may be used in a mono pro-biotic concept or a composition pro-biotic concept in obtaining or making various starters, cultured-milk, fermented and non-fermented foods, biologically active additives, bacterial preparations, makeup preparations.

The Bifidobacterium lactis 668 strain is separated from the bowels of a healthy child and deposited with VKPM (GNIIG-VKPM located at: 1, 1st Dorozhny Proezd, Moscow 113545, Russia) under No. Ac-1673.

The inventive strain survives in in-vitro conditions imitating the conditions existing in the gastrointestinal tract (GIT), in particular in the presence of 40% of bile, 0.4% of phenol, the GIT enzymes and at low pH values.

The inventive Bifidobacterium lactis 668 strain may be used in making cultured-milk products, fermented foods, such as kvass, etc., non-fermented foods, such as juices, beverages, etc., starters and biologically active additives to food. Also, the Bifidobacterium lactis 668 strain may be widely used for manufacturing makeup preparations, such as creams, lotions, nutritive masks, gels, cosmetic milk, etc.

Furthermore, the Bifidobacterium lactis 668 strain may be also used in manufacturing bacterial preparations, such as suppositories, peroral and other preparations.

The cultural, morphological and physiological-biochemical properties of the Bifidobacterium lactis 668 strain

The cells are Gram-positive, immovable, non-sporogenous, catalase-negative, anaerobic, polymorphic bacilli that are either bifurcated or thickened at 1-2 ends; they grow along the whole volume height, except for the aerobiosis area, in liquid nutritional media; when growing on a MH agarized medium in anaerobic conditions, they form small (up to 1 mm in diameter) round, colorless colonies.

The optimal incubation temperature is 37-39°C.

The Bifidobacterium lactis 668 strain ferments glucose, lactose, saccharose, maltose, salicin, xylose, arabinose, raffinose, forming acetic and lactic acids, without gas formation;

it ferments milk for 32 hours with the formation of a clump;
it actively builds up mass when growing on a maize-lactose medium (MH-1) (the MH-1 composition: a maize-milk mixture - 15 grams; peptone - 15 grams; lactose - 9 grams; ascorbic acid - 0.5 grams; triple-substituted sodium citrate - 0.6 grams; magnesium sulfate - 0.12 grams; monosubstituted potassium phosphate - 2 grams; twicesubstituted sodium phosphate - 1 gram; agar - 3 grams; distilled water - 1 liter), on a bifidum medium (BM) (the bifidum medium composition: glucose - 7.5 grams; lactose - 2.5 grams; pancreatic casein hydrolyzate - 30 grams; sodium chloride - 2.5 grams; magnesium sulfate - 0.5 grams; L-cysteine - 0.5 grams; ascorbic acid - 0.5 grams; yeastrel - 5 grams; sodium acetate - 0.3 grams; agar - 0.75 grams; distilled water - 1 liter);
builds up microbial mass to a maximum for 12-24 hours, reaching a value of 1g CFU (colony-forming unit) of 8-9;
exhibits antagonistic properties in relation to pathogenic and conditionally pathogenic microfloras (see Tables 1 and 2 given in the end of this description), namely, suppresses growth of Candida albicans and Proteus vulgaris more than 100 times, suppresses growth of Proteus mirabilis and Klebsiella pneumoniae more than 10,000 times, suppresses growth of Shigella sonnei, Citrobacter freundii, Bacillus subtilis more than 100,000 times when jointly cultivated in-vitro.

Thus, the inventive Bifidobacterium lactis 668 strain is technologically effective since it actively propagates in a nutritional medium, building up production bio-mass within short, equal to 12-24 hours, cultivation periods with high concentrations of microbial cells (lg CFU ~ 9); has acid-forming and inhibiting properties in relation to pathogenic and conditionally pathogenic microfloras, has high antioxidant activity (superoxide dismutase activity, SOD-activity is equal to 95.0 ±0.1 units / mg of protein).

Furthermore, a study of the spectrum of sensitivity to antibiotics and antibacterial preparations has found that the Bifidobacterium lactis 668 strain has the natural stability to: Aztreonam, Amikacin, Gentamicin, Kanamycin, Neomycin, Netilmicin, Polymyxin, Sisomicin, Streptomycin, Sulfanilamide, Tobramycin, Ceftazidime; it is sensitive to Laevomycetin, Ampicillin, Benzylpenicillin, Vancomycin, Doxycillin, Carbenicillin, Lincomycin, Meropenem, Oxacillin, Oleandomycin, Ofloxacin, Piperacillin, Rifampicin, Furagin, Furadonin, Furazolidone, Cefepime, Cefotaxime, Ceftriaxone, Cefuroxim.

Thus, in a number of properties and in their totality the Bifidobacterium lactis 668 strain has greater biological activity compared to the known Bifidobacterium lactis strains.

The Bifidobacterium lactis 668 strain is stable to phenol and bile, low pH values and the GIT enzymes; it has expressed antagonistic activity in relation to Proteus vulgaris, Proteus mirabilis, Klebsiella pneumoniae, Shigella sonnei, Citrobacter freundii, Bacillus subtilis; it has the natural stability to: Aztreonam, Amikacin, Gentamicin, Kanamycin, Neomycin, Netilmicin, Polymyxin, Sisomicin, Streptomycin, Sulfanilamide, Tobramycin, Ceftazidime; it has good technological effectiveness (i.e., the strain's capability of growing in a big titer equal to 1g CFU-9 on selected media and preserving itself well in cultured-milk products during a required prolonged keeping time) and expressed antioxidant activity.

The Bifidobacterium lactis 668 strain's capability of growing in a big titer on a nutritional medium for short cultivation periods, its antagonistic, acid-forming, antioxidant activities, capability of preserving itself for a long time in cultured-milk products as well as other useful properties enable to use the strain in compositions of food products and dietotherapy preparations.

### Description of preferred embodiments

### Example 1. Use of the Bifidobacterium lactis 668 strain in compositions of biological substances, in particular in compositions of biologically active additives.

1 passage: the lyophilized strain of Bifidobacterium lactis 668 in the quantity of ~0.1 grams of dry biomass is introduced in vials with a nutritional bifudum medium and is titrated by the method of tenfold dilutions to 10⁻⁹. Incubation is carried out at 37-38°C until visible growth in last dilutions is obtained.
2 passage: a grown culture from 10⁻⁷⁻⁹ dilutions in a volume up to 10% is introduced in a nutritional bifidum medium and cultivated at 37-38°C for 12-24 hours until a dense growth with a visible aerobiosis area is formed.
3 passage: the obtained biomass in a quantity up to 10% of the volume is introduced in an accumulation medium and cultivated for 12-24 hours at 37-38°C until biomass with the live bifidus bacteria content at least 108 CFU/mL is obtained.

In the result, a biologically active additive is obtained, which is a suspension of Bifidobacterium lactis 668 cells with the titer at least 108 CFU/mL. If necessary, a higher-titer concentrate of bifidus bacteria may be obtained.

### Example 2. Use of the Bifidobacterium lactis 668 strain for making non-fermented food products.

1 passage: the lyophilized strain of Bifidobacterium lactis 668 in the quantity of ~0.1 grams of dry biomass is introduced in vials with a nutritional bifudum medium and is titrated by the method of tenfold dilutions to 10⁻⁹. Incubation is carried out at 37-38°C until visible growth in last dilutions is obtained.
2 passage: a grown culture from 10⁻⁷⁻⁹ dilutions in a volume up to 10% is introduced in a nutritional bifidum medium and cultivated at 37-38°C for 12-24 hours until a dense growth with a visible aerobiosis area is formed.
3 passage: the obtained biomass in a quantity up to 10% of the volume is introduced in an accumulation medium and cultivated for 12-24 hours at 37-38°C until biomass with the live bifidus bacteria content at least 108 CFU/mL is obtained.

The obtaining of a non-fermented food product: grown and, if necessary, concentrated biomass is introduced in food raw stock (milk, juices, etc.), mixed and dispensed in containers. In the result, a product with the concentration of live bifidum bacteria at least 106 CFU/mL is obtained.

### Example 3. Making a cultured-milk product for treatment and prophylaxis purposes with the use of the Bifidobacterium lactis 668 strain as a component of starting cultures for making fermented cultured-milk products.

1 passage: the lyophilized strain is introduced in a cultivation bifudum medium and is titrated by the method of tenfold dilutions to 10⁻⁹. Incubation is carried out at 37-38°C until visible growth in last dilutions is obtained.
2 passage: a grown culture of the Bifidobacterium lactis 668 strain from last dilutions is introduced in an accumulation medium and cultivated at 37-38°C until biomass with the live bifidus bacteria content at least 108 CFU/mL is obtained.
3 passage: the obtained biomass in a quantity up to 10% of the volume is introduced in an accumulation medium and cultivated for 12-24 hours at 37-38°C until biomass with the live bifidus bacteria content at least 108 CFU/mL is obtained.

The obtaining of a cultured-milk food product: the obtained biomass of the Bifidobacterium lactis 668 strain in a quantity up to 10% is introduced in a pasteurized milk mixture at 37-38°C together with other starting cultures used at normal concentrations and cultivated until a clump (pH=4.5-4.6) is formed.

The obtained product with the concentration of live bifidum bacteria of Bifidobacterium lactis 668 at least 106 CFU/mL is cooled down to 22°C, dispensed in containers and additionally cooled down to 6°C. The number of passages may be increased, depending on a production volume.

### Example 4. Use of the Bifidobacterium lactis 668 strain for making bacterial preparations.

The strain culture from the 1st or the 2nd passage (Example 1) is introduced in a nutritional bifidum medium and cultivated at 37-38°C until biomass of bifidum bacteria with the content of live cells at least 108 CFU/mL is obtained. Protective drying media (e.g., a saccharose-gelatin medium, skimmed milk). The obtained suspension is dispensed in ampoules, or flasks, or other containers, frozen at minus 40-45°C and lyophilized from the frozen state in vacuum ovens. The obtained preparation contains at least 108 live cells of Bifidobacterium lactis 668 bifidum bacteria in 1 gram. By forming doses of adequate consumption level of pro-biotic microorganisms in a day, which are equal to at least 5x10⁸ - 5x10¹⁰ CFU (for bifidum bacteria), it is possible to use the obtained preparation as a bacterial preparation for a pro-biotic.

### Example 5. Use of the Bifidobacterium lactis 668 strain for making makeup preparations.

The strain culture from the 1st or the 2nd passage (Example 1) is introduced in a nutritional bifidum medium and cultivated at 37-38°C until biomass of Bifidobacterium lactis 668 bifidum bacteria with the content of live cells at least 108 CFU/mL is obtained. The obtaining of makeup preparations: the grown biomass (or, if necessary, a concentrate) of the 668 bifidum bacteria strain is introduced into a composition of a makeup preparation.

### Example 6. Use of the Bifidobacterium lactis 668 strain for making a fermented product.

### The strain culture from the 1st or the 2nd passage (Example 1) is introduced in a nutritional bifidum medium and cultivated at 37-38°C until biomass of bifidum bacteria with the content of live cells at least 108 CFU/mL is obtained.

In order to make a fermented product of, e.g., kvass type, the grown biomass (or, if necessary, a concentrate) of bifidum bacteria of the Bifidobacterium lactis 668 strain is introduced in a prepared substrate (water, sugar, honey, wort, ascorbic acid) together with Lactobacillus plantarum and Lactobacillus fermentum, mixed and cultivated at 37°C until a beverage with the titratable acidity of 65-85°T is obtained. Then the beverage is cooled down and dispensed in bottles. In the result a ready-made product for treatment and prophylaxis is obtained, which comprises at least 107 live pro-biotic microorganisms per 1 mL.

### Antagonistic activity of the Bifidobacterium lactis 668 strain (as claimed) and the Bifidobacterium lactis 420 strain (prototype) in relation to pathogenic microorganisms

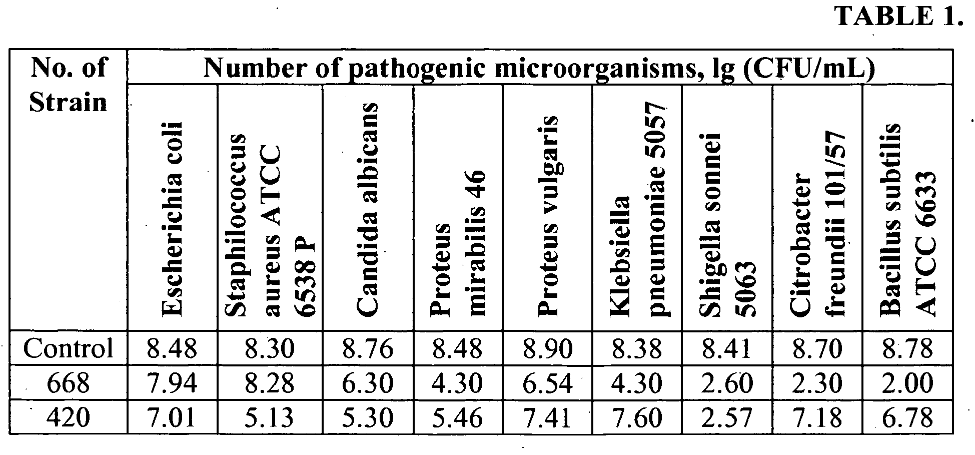

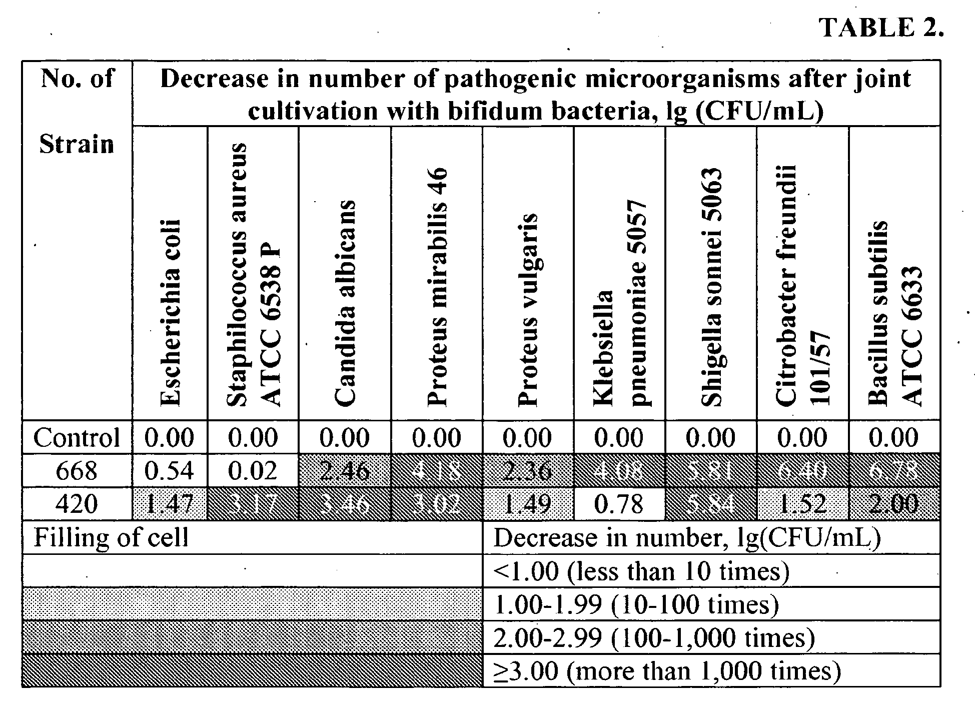

## Claims

1. The bifidum bacteria Bifidobacterium lactis 668 strain, VKPM Ac-1673, used for making cultured-milk, fermented and non-fennented food products, biologically active additives, bacterial preparations and makeup preparations.

## Patentansprüche

1. Der Bifidum-Bakterien-Stamm Bifidobacterium lactis 668, VKPM Ac-1673, der für die Herstellung von durch Bakterienkulturen fermentierter Milch, fermentierten und nicht-fermentierten Lebensmitteln, biologisch aktiven Zusatzsubstanzen, bakteriellen Präparaten und Kosmetikpräparaten verwendet wird.

## Revendications

1. La souche des bactéries bifides Bifidobacterium lactis 668, VKPM Ac-1673, qui est utilisée pour la production de produits de lait fermenté par une culture bactérienne, de produits alimentaires fermentés ou non fermentés, d'additifs biologiquement actives, de préparations bactériennes et de préparations cosmétiques.
